# EUROPEAN PATENT APPLICATION

(11) **EP 2 083 071 A1**
(43) Date of publication of application: **29.07.2009**
(21) Application number: 08450009.9
(22) Date of filing: 25.01.2008
(51) Int. Cl.: C12N 5/00

(54) **Medium for propagating and expanding stem cells**

(71) Applicant: Cell Med Research GMBH, 3502 Krems-Lerchenfeld (AT)
(72) Inventor: Rubiolo, Cristina, 1220 Vienna (AT); Stadelmann, Silke, 1160 Vienna (AT)
(74) Representative: Sonn & Partner Patentanwälte

(57) **Abstract**

The present invention relates to a growth medium for in vitro stem cell expansion comprising:

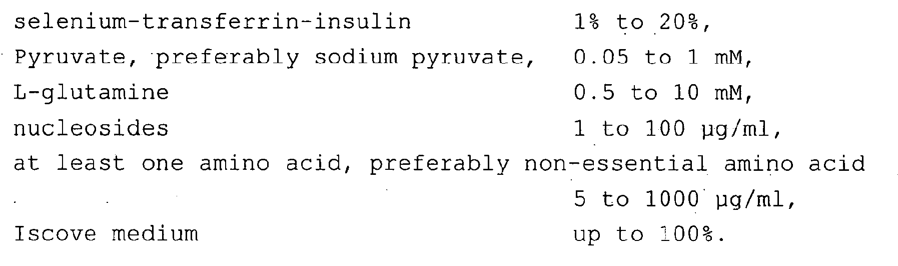

## Description

The present invention relates to a medium for propagating and expanding stem cells.

Stem cells are commonly defined as cells, which exist for the lifetime of an organism and are able to undergo symmetric and/or asymmetric divisions, to give rise to further stem cells (for preservation of the stem cell pool) and to more differentiated cells with defined life-time (for organ-specific functions). Due to this unique property they are ideal vehicles for somatic gene therapy. Indeed, once stably transfected, they would maintain the transgene for the life-time of the tissue and the organism, and would carry the transgene expression into the differentiated cells. Stem cells may be totipotent (e.g. embryonic stem cells), pluripotent (e.g. hematopoietic stem cells, neural glial stem cells, hepatocyte stem cells, chondrocytic stem cells) or unipotent (e.g. keratinocytic stem cells, muscle precursor cells, tracheal epithelial precursor cells).

It is an object of the present invention to provide a growth medium for the in vitro expansion of stem cells.

The present invention relates to a growth medium for in vitro (adult) stem cell expansion comprising:

| | |
|---|---|
| selenium-transferrin-insulin | 1% to 20%, |
| Pyruvate, preferably sodium pyruvate, | 0.05 to 1 mM, |
| L-glutamine | 0.5 to 10 mM, |
| Nucleosides | 1 to 100 µg/ml |
| at least one amino acid, preferably non-essential amino acid | 5 to 1000 µg/ml, |

Iscove's modified Dulbecco's medium (IMDM) up to 100%.

The medium according to the present invention can be used to expand and/or cultivate stem cells while maintaining intact their differentiation potentiality towards specific cell-types. The transferrin present in the medium according to the present invention is of human origin, whereby the insulin is synthetically produced, preferably with recombinant methods.

Stem cells and in particular adult stem cells can usually not be cultured with (basic) IMDM. However, it could be shown that the addition of further components results in a medium, which can be used to expand and propagate adult stem cells.

The adult stem cells, which can be propagated with the medium according to the present invention may be obtained preferably from cord-blood, placenta or amniotic fluid. Thereby the inventive medium ("HMF-SCm") shows several advantages in comparison to similar products known in the art:
1. High rate of pure stem cells/erythroid cells harvesting
2. Absence of sera, sera derivates (i.e. albumin) or plasma
3. Suitable for different type of SCs
4. Suitable for different cell types in combination with a corresponding mixture of growth factors (the composition of said mixtures depends on the type of cells to be differentiated and is known to the person skilled in the art)
5. No need of enrichment steps
6. No need of cell-derived feeding layers
7. No need to feed the cells every day
8. High and stable cell viability
9. High rate of cryo-preservation with different freezing media: cell phenotype and functionality mantained after repeated (till 3 times) cycles of freezing/thawing
10. High transdifferentiation potentialities
11. Stable undifferentiate state of SCs and erythroblasts with high cell proliferation
12. Absence of phenol red and therefore suitable for studies with minimal quantity of steroid hormones
13. Medium stability at -20°C and -180°C
14. Medium stability at 4°C

HMF-SCm fulfils quality criteria, which result in:
1. A strong improvement of stem cells expansion to quantities suitable for large trans-differentiation studies from human sources (50-100*10⁶ cells, according from the starting amount of CB-/placenta-/amniotic fluid-derived cells);
2. A high-rate stem cells expansion in a very defined short-time (e.g. 10 days);
3. *Re-vivo* use

All these characteristics make HMF-SCm particularly indicated for clinical study and whenever an *ex-vivo* expansion of SCs is required, before *re-vivo* reinjection.

The medium of the present invention can be used to expand and for the trans-differentiation of stem cells in general and in particular of hematopoietic stem cells, mesenchymal stem cells and amniotic stem cells.
**1. Hematopoietic stem cells** (HSCs), which can be isolated from bone marrow, peripheral blood, placenta and cord-blood and subsequently expanded and trans-differentiated into both white and blood line. HSCs therapy may play a major role in the cure of several cancers, such as acute and chronic leukemias, myelodysplastic syndromes, aplastic anemia, myelo- and lymphoproliferative disorders, phagocyte disorders and other genetic disorders, such as congenital thrombocytopenia. A major thrust of basic HSCs research, since the 1960s, has been identifying and characterizing these SCs. Because HSCs look and behave in culture like ordinary white blood cells, this has been a difficult challenge and this makes them difficult to identify by morphology (size and shape). Even today, scientists must rely on cell surface proteins, which serve, only roughly, as markers of white blood cells. The challenge is formidable as about 1 in every 10,000 to 15,000 bone marrow cells is thought to be a stem cell. In the blood stream the proportion falls to 1 in 100,000 blood cells. HCSs are ultimately responsible for the constant renewal of blood. The transplantation of bone marrow to restore a healthy blood system in leukemia patients is limited by the unavailability of HSCs in quantity and purity that are crucial for successful transplantation. Because of their relative rarity (one in every 10,000 bone marrow cells) and the difficulty of separating them from other components of the blood, bone marrow transplants are generally impure. Obtaining purified hematopoietic stem cells is a major challenge, and purification in a clinical setting is expensive and difficult.
   HMF-SCm supports, within 10 days, a rate of HSCs harvesting of about 60% from the initial total cells population, derived from cord-blood or placenta. This means that one recover around 50-100 *10⁶ cells, which can be further trans-differentiated or expanded.
**2. Mesenchymal stem cells (MSCs).** Several studies have demonstrated that multipotent MSCs, under specific permissive conditions, can be induced to differentiate into bone, adipose, cartilage, muscle, and endothelium. In particular, MSCs, because of their unique immunologic characteristics that suppress lymphocyte proliferation *in vitro* and prolong skin graft survival *in vivo*, may survive longer in a xenogeneic environment. Data from preclinical transplantation studies suggested that MSCs infusions not only prevent the occurrence of graft failure but also have immunomodulatory effects. Nevertheless, MSCs are a rare population (approximately 0.001-0.01%) of adult human bone marrow and of the adult peripheral blood and their number significantly decreases with age. Moreover, MSCs are also relatively few in term cord blood. In this context, a recent study showed that a population of MSC-like cells exists within the umbilical vein endothelial/sub endothelial layer. However, term CBs compared with preterm ones are a poor source of MSCs. As shown by Miao et al., (2006), it is possible to isolate a population of pluripotent cells from the human term placenta, a temporary organ with fetal contributions that is discarded postpartum and that exhibits many markers common to bone marrow mesenchymal stem cells (BMSCs). Placental MSCs (PMSCs) may therefore provide an ethically uncontroversial and easily accessible source of multipotent cells for future experimental and clinical applications.
   Using routine cell culture techniques, PMSCs can be successfully isolated and expanded in vitro. Although the initial cell culture consists of both fibroblastoid and non-fibroblastoid cell types, only the fibroblastoid population remain after enzymatic digestion and passaging. In their undifferentiated state MSCs are spindle-shaped and resemble fibroblasts. There are no markers, which specifically and uniquely identify MSCs and therefore they are defined by their immunophenotypic profile as well as by their characteristic morphology; indeed, while MSCs express neither haemopoietic markers (e.g. CD45, CD34, CD14) nor endothelial markers (e.g. CD34, CD31, vWF), they do express a large number of adhesion molecules (e.g. CD44 and integrins), some stromal cell markers (e.g. SH-2, SH-3 and SH-4) and some cytokine receptors (e.g. IL-1R, TNF-R).
   Currently, the presence of MSCs in umbilical CB is still contested, and in reports that document isolation of such cells, low yield and inter-individual variation are consistently encountered limitations. On the contrary, PMSCs can be easily isolated and expanded without morphological and characteristic changes in medium supplemented only with FBS. Therefore, the placenta may prove to be an attractive and rich source of MSCs. The presence of stem cells in the placenta could have tremendous implications. The initial data on the differentiation capabilities of PMSCs are promising, and there may be important therapeutic uses for these cells. Along with the ease of accessibility, lack of ethical concerns, and abundant cell number, PMSCs may be an attractive, alternative source of progenitor or stem cells for basic research.
   HMF-SCm suppports a rate of harvesting of MSCs of about 40-50% from the initial total cells population, derived from cord-blood or placenta within 10 days. This means that one can recover around 40-80*10⁶ cells that can be further trans-differentiated or expanded. Moreover, this approach eliminates at once the problems related to low yield, individual variance and requirement of serum for cell expansion.
**3. Amniotic stem cells.** Amniotic epithelial cells develop from the epiblast by 8 days after fertilization and prior to gastrulation, opening the possibility that they might maintain the plasticity of pre-gastrulation embryo cells. Amniotic epithelial cells isolated from human term placenta express surface makers normally present on embryonic stem and germ cells. In addition, amniotic epithelial cells express the pluripotent stem cell specific transcription factors octamer-binding protein 4 (Oct-4), and nanog. Under certain culture conditions, amniotic epithelial cells form spheroid structures, which retained stem cell characteristics. Amniotic epithelial cells do not require other cell-derived feeder layers to maintain Oct-4 expression, do not express telomerase and are non-tumorigenic upon transplantation. Based on immunohistochemical and genetic analysis, amniotic epithelial cells have the potential to differentiate to all three-germ layers-endoderm (liver, pancreas), mesoderm (cardiomyocytes), and ectoderm (neural cells) *in vitro.* Amnion derived from term placenta, following live birth, may be a useful and non-controversial source of stem cells for cell transplantation and regenerative medicine.

The medium (HMF-SCm) of the present invention is particularly suited to culture human hematopoietic, mesenchimal and fibroid-like stem cells from cord-blood, placenta and amniotic fluid as well as to culture human erythroid cells from cord-blood and placenta without the need of intermediate enrichment steps, such as the use of magnetic beads. Hematopoietic cells as well as erythroid cells are cultured in absence of feeding layers and/or a coating mix. The mesenchimal/fibroid-like stem cells are cultured in absence of feeding layers but on a coating mix, designed by Cell Med. The rate of hematopoietic stem cells harvesting is 60% of the initial population after 10 days. The rate of mesenchimal/fibroid-like stem cells harvesting is 40% of the initial population after 10 days. The rate of erythroid precursors harvesting is 95% of the initial population after 10 days. The cells are cultured in presence of different growing factors (GFs) mixtures, developed in order to guarantee the maximum harvesting rate of the selected cells. All cell types cultured in HMF-SCm are stable upon cryopreservation in glycerol or DMSO between -80 und -180°C. Cells can be kept in culture for max 20 days (stem cells) or max 60 days (erythroid cells). After 10-12 days about 50-100 *10⁶ stem cells or erythroid cells can be harvested. Erythroid cells as well as stem cells must be fed every second day. After 20 days in culture, the erythroblasts must be ficolled every 5 days. Erythroid and stem cells can be terminally differentiated, following different protocols. Cell viability is always ≥ 85%.

According to a preferred embodiment of the present invention the medium comprises further LDL, preferably human LDL, in an amount of 1 to 100 µg/ml. In the presence of LDL, the cells are more stable and they do not spontaneously differentiate or die. Furthermore, the addition of LDL results in a better proliferation of the cells.

According to another preferred embodiment of the present invention the medium comprises further an iron salt, preferably ferrous sulphate, in an amount of 1 to 100 nM.

According to the present invention a medium comprising the following components is particularly preferred:

| | |
|---|---|
| Iscove medium | to 500 mL |
| Selenium-Transferrin-Insulin | 50 mL |
| Sodium Pyruvate | 0.1 mM |
| L-Glutamine | 2 mM |
| the four DNA-specific nucleosides) | 10 µg/mL |
| Non essential amino acids | 100 µg/mL |

According to the most preferred embodiment of the present invention the medium comprises:

| | |
|---|---|
| Iscove medium | to 500 mL |
| Selenium-Transferrin-Insulin | 50 mL |
| human LDL | 40 µg/mL |
| Sodium Pyruvate | 0.1 mM |
| L-Glutamine | 2 mM |
| Iron Sulphate | 40 nM |
| Nucleoside | 10 µg/mL |
| Non-essential amino acids | 100 µg/mL |

To the medium according to the present invention the following group consisting of SCF, GM-SCF, TPO and Flt-3 of growing factors must be added(the amount of growth factor added may vary from 1 to 200µg/mL). It is particularly preferred to add to the medium 100 µg/mL SCF, 3.5 µg/mL GM-SCF, 7 µg/mL TPO and/or 20 µg/mL Flt-3.

Another aspect of the present invention relates to the use of a medium according to the present invention for cultivating/expanding hematopoietic, amniotic and/or mesenchymal stem cells.

Yet another aspect of the present invention relates to a method for cultivating hematopoietic, amniotic and/or mesenchymal stem cells comprising the steps:
- providing a stem cell source, and
- incubating said source with medium according to the present invention.

The present invention is further illustrated in the following figures and example, however, without being restricted thereto.
Fig. 1 shows cell phenotypes of HSC, derived from CB, and cultured in different media and GF mixtures.
Fig. 2 shows hemoglobin accumulation by HSCs differentiated into erythrocyte in different media and with different cytokines (GF1 vs GF2).

### EXAMPLE:

### Materials and Methods

### SCs expansion

Term (38-40 weeks gestation) placentas and CBs or amniotic fluid were obtained from healthy donor mothers. CB-derived cells were ficolled and put in culture. The placenta tissue was washed, mechanically minced, enzymatically digested with 0.25% trypsin-EDTA, filtered and centrifuged. The cells were finally collected and the erythrocytes lysed. The homogenate was subsequently pelleted, resuspended in complete medium and cultured at 37 °C with a water-saturated atmosphere and 6.5% CO₂. Medium was replaced every second day.

The growth kinetics of SCs is assessed every day over 2 weeks.
1. HMF-SCm:

| | |
|---|---|
| Iscove medium | to 500 mL |
| Selenium-Transferrin-Insulin | 50 mL |
| human LDL | 40 µg/mL |
| Sodium Pyruvate | 0.1 mM |
| L-Glutamine | 2 mM |
| Iron Sulphate | 40 nM |
| Nucleoside | 10 µg/mL |
| Non-essential a.a. | 100 µg/mL |

2. HMF-GF:

| | |
|---|---|
| SCF | 100 µg/mL |
| GM-SCF | 3.5 µg/mL |
| TPO | 7 µg/mL |
| Flt-3 | 20 µg/mL |

3. MASC:

| | |
|---|---|
| Iscove medium | To 15 mL |
| Collagen I+IV | 0.5% |
| Fibronectin | 0.1% |
| bFGF | 5 µg/mL |
| VEGF | 0.3 µg/mL |
| EGF | 1 µg/mL |
| SCF | 50 µg/mL |
| Gelatin | 1% w/v |

### SCs transdifferentiation

The differentiation potential of HSCs towards erythroid cells has been investigated as follows.

After 2 days expansions, the cells were differentiated into erythroid precursors upon incubation with 1 U/mL human EPO, 100 ng/mL SCF, 10⁻⁶ M Dex and 40 ng/mL IGF-1 and after 6 days were further differentiated into fully mature erythrocyte upon incubation with 10 U/mL Epo, 10 ng/mL insulin, 3x10⁻⁶ M ZK112.993, and 1 mg/mL iron-saturated human transferrin. Alternatively, cells were cultured 4 days in HMF-SCm + HMF-GFs and terminally differentiated into erythrocytes by incubation with 10 U/mL Epo, 15 ng/mL insulin, 100 ng/mL IL-3, and 1.5 mg/mL iron-saturated human transferrin.

Other differentiation potentials of SCs were investigated in cell type-specific media.

Differentiation into endothelial-like cells was induced by culturing semi-confluent cells in presence of 2% fetal calf serum and 50 ng/mL vascular endothelial growth factor (VEGF), while the endothelial phenotype is detected by staining with antibody against Von Willebrand Factor (vWF).

For neuron-like or astrocyte-like cells differentiation, cells were cultured in the presence of 2% dimethyl sulfoxide (DMSO) and 100x10⁻⁶ M butylated hydroxyanisole (BHA). The differentiated cells were identified by staining with antibodies against neuron specific enolase (NSE) and glial fibrillary acid protein (GFAP).

The stem cells differentiation into smooth muscle cells was induced by culturing them in the Quantum 212 (PAA) plus SupplementalPack (PromoCell, C-39262), whereas their differentiation into skeletal muscle cells is induced by culturing them in the Quantum 212 (PAA) plus SupplementMix (PromoCell, C-39360).

SmMC were detected by ASMA, smoothelin, calponin, caldesmon and SM22. SkMC were detected by anti-skeletal myosin.

Stem cells were cultured in the Quantum 212 plus SupplementMix (PromoCell, C-39262) or in the Quantum 212 plus SupplementMix (PromoCell, C-39360) for about 2 weeks, washed with PBS, and incubated with a bath solution consisting of 155 mM NaCl, 4.5 mM KCl, 2 mM CaCl₂, 1 mM MgCl₂, 5 mM glucose, and 10 mM Hepes. Contraction was elicited by incubation with 10⁻⁵M carbachol, and relaxation was subsequently elicited by addition of 10⁻⁴M atropine. The ability of differentiated muscle cells to contract in response to 10⁻¹¹M, 10⁻⁸M, and 10⁻⁵M carbachol was evaluated together with their ability to respond to atropine, investigated by incubation in 10⁻⁴M atropine for 45' followed by addition of carbachol through the use of the 3D collagen culture kit (Chemicon, ECM675).

The stem cells were induced to differentiate into myocytes by culturing them in D-MEM: M199 (4:1), supplemented with 15% horse serum, 5% FBS, 5mM of sodium butyrate (or 2% DMSO) and 10 mM of BDM (2,3-butanedione monoxime; Sigma B0753-100G), in order to impair myofibrillogenesis. The myocytes were detected by desmin (Chemicon, MAB3430) and connexin 43 (Chemicon, AB1728).

### FACS

Stem cells were detected by the evaluation of the following markers expression:
ABC-G2, SSEA-4, CD34, CD117, CD133, CD45, CD9, CD166, CD10, CD3, CD19, CD3, CD44, CD105, CD73, CD13, CD90, HLA-DR, HLA-ABC, CD29 and CD14.

Erythroid cells were detected by the evaluation of the following markers expression:
CD71, GPA, CD34, CD36, CD117, CD14, CD3 and CD45,

### Hemoglobin accumulation

To quantify the hemoglobin accumulation, every day 50 µL of cell suspension were applied in triplicate to a 96 well plate and centrifuged for 5' at 1200 rpm, washed once with PBS, eventually resuspended in 30 µL of milliQ water and kept at -80°C.

At the end of each set of experiments, the plates were stained as described in the following protocol: the plates are thawed and 125 µL of citrate phosphate buffer (IV of 0.1 M citrate mixed with 2V of Na₂HPO₄) mixed with 0.5 mg/mL O-phenylene-diamide and H₂O₂ (1µL/mL) were added to each well.

As soon as the reaction mix in the wells becomes yellow, the reaction was stopped by adding 25 µL H₂SO₄ (8 N) to each well. The extinction was measured by the ELISA photometer at 492 nm and counter-measured at 620 nm, a mean to correct artifacts, due to the presence of particles and/or bubbles. Values were obtained from triplicate determinations, averaged and normalized to cell number and cell volume.

### Proliferation assay

Cells were thawed and co-cultured at a final concentration of 1*10⁴ cells/well in a 96-well U-bottom plate in HMF-SC + HMF-GFs, supplemented with 10% AlamarBlue at a final volume of 150 µL. Proliferation caused reduction of the dye changing from oxidized (non-fluorescent, blue) form to reduced (fluorescent, red) form. For 2-7 days, every 4-12h, the absorbance was measured in a plate-reader at 595 nm with reference wavelength at 620 nm.

### Western blot

### Cytoplasmatic fraction

The cells were lysed in Frackelton buffer for 20' in ice and centrifuged at 1000g 10' at 4°C.

After collecting the supernatant, the protein concentration was measured with the Bradford assay and blue sample buffer is added to each sample in a ratio of 4:1.

### Nuclear fraction

The cells were lysed in NP40 buffer for 5' in ice and centrifuged at 800g 3' at 4°C.

After collecting and resuspending the pellet, the protein concentration was measured with the Bradford assay and blue sample buffer was added to each sample in a ratio of 4:1.

After preparing a polyacrylamide gel, the proteins were run and blotted on a nitrocellulose membrane.

Thereafter, the membrane was washed in TBS-T and incubated at first 1h in the blocking solution, made up of TBS-T 1X, added up with 0.05% Tween-20 and milk 5% and then in the primary antibody, diluted in TBS-T w/o milk, added up of 3% BSA and 0.02% NaN₃, O/N.

Afterwards the membrane was washed 3X in TBS-T and incubated with the secondary antibody for 40' at RT.

Finally the protein expression was detected with the ECL kit.

### Immunofluorescence (IF)

The cells were washed with cold PBS 2X 5'; fixed with MetOH + 5 mM EDTA (-20°C), for 7'; washed with cold PBS pH=8 2X 5' and with Q/W 1X 5', before permeabilizing them with Triton 0.05% 10' at RT and washing them with Q/W 3X 5'.

Thereafter the cells were incubated with a blocking solution made of 0.2% gelatin, 0.3% Triton, 50 mM NH₄Cl and 10% horse serum in 1X CB for 30' at 37°C and with the primary antibody, diluted in 0.2% gelatin, 0.3% Triton, 50 mM NH₄Cl and 10% horse serum in 1X CB for 1h at 37°C, before washing with Q/W 3X 5' and incubating with the secondary antibody, diluted in 0.2% gelatin, 0.3% Triton, 50 mM NH₄Cl and 10% horse serum in 1X CB for 1h at RT. Thereafter the cells were washed with Q/W 3X 5'; incubated with a blocking solution made of 10% horse serum, 1% BSA, 0.02% NaN₃ in PBS pH=8 for 30' at RT and washed with cold TBS pH=8 3X 5'. Finally the cells were incubated with the primary antibody diluted in 1% BSA in TBS pH=8 (40 µL) O/N, washed with cold TBS pH=8 3X 5', incubated with the secondary antibody (1:1500) diluted in 1% BSA in TBS pH=8 for 1h at RT and washed with cold TBS pH=8 3X 5'.

At the end, the coverslip was mounted with ProLong Antifade kit.

### Model of heart failure

Myocardial neovascularization was assessed by capillary density; LV remodelling was evaluated by the percent of fibrosis area. To evaluate LV function, transthoracic echocardiography was performed immediately before as well as 5 and 28 days after MI. Under general anesthesia with ketamine and xylazine, LV end-diastolic and end-systolic dimensions (LVEDD and LVESD, respectively) and fractional shortening (FS) were measured at the mid-papillary muscle level. Regional wall motion score (RWMS) was evaluated.

Immediately after the final echocardiography on day 28, the rats underwent cardiac catheterization for more invasive and precise assessment of global LV function. A 2.0F micromanometer-tipped conductance catheter was inserted via the right carotid artery into the LV cavity. LV pressure and its derivative (LV dP/dt) were continuously monitored with a multiple recording system.

Heart rate, LV end-diastolic pressure (LVEDP), LV ejection fraction (EF), and the maximum and minimum LV dP/dt (+dP/dt and - dP/dt, respectively) were recorded continuously for 20 minutes. All data were acquired under stable hemodynamic conditions.

Upon killing, only those rats with infarct size >25% of the left ventricle area, were included in the study.

### Tissue Harvesting

All rats were killed 28 days after transplantation with potassium chloride. At necropsy, hearts were sliced in a broadleaf fashion into 4 transverse sections from apex to base, embedded in OCT compound, snap-frozen in liquid nitrogen, and stored at - 80°C.

Rat hearts in OCT blocks were sectioned, and 5 µm serial sections were collected on slides, followed by fixation with 4% paraformaldehyde at 4°C for 5' and stained immediately.

### Morphometric Evaluation of Capillary Density and Infarct Size

Histochemical staining with isolectin B4 was performed, and capillary density wasmorphometrically evaluated by histological examination of 5 randomly selected fields of tissue sections, recovered from segments of LV myocardium, subserved by the occluded left anterior descending coronary artery. Capillaries were recognized as tubular structures positive for isolectin B4. To elucidate the severity of myocardial fibrosis, Masson trichrome staining was performed on frozen sections from each tissue block, and the stained sections were used to measure the average ratio of fibrosis area to entire LV area (percent fibrosis area). Masson trichrome staining was performed with a kit (Dako).

### Morphological and Histological Studies

After completion of all measurements, the heart was arrested with potassium chloride and rapidly excised. The coronary arteries were perfused with 100 mL 10% formaldehyde, and the heart was fixed in diastole position with an intraventricular pressure of 30 mm Hg in formaldehyde solution. The fixed heart was sliced into 5 mm thick slices and photographed. Cross-sectional areas of the ventricular muscle, scar area, and scar thickness were measured. A cube of tissue of approximatively 5 mm from the center of the infarct zone were embedded in paraffin and cut into 10 µm sections. Serial cutting sections were used for immunohistochemical studies to localize the transplanted cells, infiltrated cells, and contractile protein positive cells.

For angiogenesis analysis, the myocardial biopsies were sectioned and immunohistochemically stained for factor VIII and α-smooth muscle actin. The number of large blood vessels (both factor VIII and smooth muscle actin positive) and capillaries (factor VIII positive only) were counted in a blinded fashion and compared between the groups.

### Results

### SCs expansion after 10 days culture from fresh isolated cells from placenta and cord-blood

Cells isolated from cord blood (CB) or placenta, were cultured in presence of HMF-GF mix, on MASC coated dishes for 1 day. After 24h, the non-adherent cells (HSCs) were transferred into non-coated dishes and cultured for additional 9 days. The adherent cells (MSCs/Fibroid-like SCs) were further cultured on coated dishes. The medium + GFs was replaced every second day.

After 10 days, about 80*10⁶ HCSs and about 50*10⁶ MSCs/Fibroid-like SCs were harvested and their phenotype was analysed by FACS.

Non-adherent cells derived from placenta and cultured in uncoated dishes were positive for both HSCs markers and MSCs markers. Adherent cells derived from placenta and cultured in coated dishes were mildly positive for HSCs markers and high positive for MSCs markers. All these data together argue that placenta is a better source for MSCs than for HSCs and that non adherent cells, derived from placenta still express many MSCs markers, along with HSCs ones.

**Table 1. Analysis of the phenotype of cells derived from placenta and cultured with HMF-SC, added up with HMF-GFs mix, in presence (MSC/fibroid-like SCs) and in absence (HSCs) of the coating solution according to the present invention.**

| | | **non adh.** | **adh** |
|---|---|---|---|
| HSC | ABC-G2 | 21.7 | 12.2 |
| | ABC-G2+CD34 | 20.9 | 11.5 |
| | ABC-G2+CD133 | 21.4 | 12.2 |
| | CD34 | 45.9 | 18.9 |
| | CD133 | 24.4 | 16.4 |
| | CD34+CD133 | 22.7 | 13.8 |
| | CD117 | 43 | 33 |
| | CD34+CD29 | 20.5 | 18.8 |
| | CD34+CD105 | 22.7 | 14.8 |
| | CD34+ HLA-DR | 9.5 | 5.5 |
| | CD34+CD44 | 23.6 | 9.8 |
| | CD34 + HLA-ABC | 23.8 | 16.8 |
| MSC | CD105 | 30 | 48.6 |
| | HLA-ABC | 60.5 | 73.6 |
| | CD105 + HLA-ABC | 20.6 | 45.9 |
| | HLA-DR + HLA-ABC | 4.7 | 4.5 |
| | CD105 + HLA-DR | 7.4 | 4.8 |
| | CD44 | 94.7 | 98.4 |
| | CD29 | 41.3 | 55.2 |
| | CD44+CD29 | 40.5 | 41 |
| | HLA-DR+CD29 | 6.1 | 4.4 |
| | HLA-DR+CD44 | 5.4 | 5.1 |
| | HLA-DR | 10.1 | 9.6 |
| | CD3 | 7 | 2.8 |
| | CD14 | 15 | 16.7 |
| | CD19 | 5.5 | 5.5 |

T-cell contamination (CD3⁺ cells) was lower than 7%; B-cell contamination (CD19⁺ cells) was lower than 6% and monocyte contamination (CD14⁺ cells) was lower than 17%.
HLA-DR⁺ cells were less than 10.5% (Table 1).

Non-adherent cells derived from CB and cultured on uncoated dishes were strongly positive for HSCs markers but almost completely negative for MSCs markers. Adherent cells derived from CB and cultured in coated dishes were almost negative for HSCs markers and strongly positive for MSCs markers. All these data together argue that CB is an excellent source for MSCs as well as for HSCs. Moreover, CB-derived HSCs accumulate in the non-adherent portion and the MSC/fibroid-like SCs accumulate in the adherent portion. Therefore, CB is a suitable source for both kind of SCs and it support as well a good separation rate between the 2 portions by adherence.

T-cell contamination (CD3⁺ cells) was lower than 7.5%; B-cell contamination (CD19⁺ cells) was lower than 6% and monocyte contamination (CD14⁺ cells) was lower than 17%. HLA-DR⁺ cells were less than 11% (Table 2).

**Table 2. Analysis of the phenotype of cells derived from CB and cultured with HMF-SC, added up with HMF-GF mix, in presence (MSC/fibroid-like SCs) and in absence (HSCs) of the coating solution according to the present invention.**

| | | **non adh.** | **adh** |
|---|---|---|---|
| HSC | ABC-G2 | 31.7 | 12.2 |
| | ABC-G2+CD34 | 30.9 | 11.5 |
| | ABC-G2+CD133 | 31.4 | 12.2 |
| | CD34 | 45.9 | 18.9 |
| | CD133 | 44.4 | 16.4 |
| | CD34+CD133 | 42.7 | 13.8 |
| | CD117 | 63 | 33 |
| | CD34+CD29 | 30.5 | 18.8 |
| | CD34+CD105 | 28.7 | 14.8 |
| | CD34+ HLA-DR | 9.5 | 5.5 |
| | CD34+CD44 | 33.6 | 9.8 |
| | CD34 + HLA-ABC | 43.8 | 16.8 |
| MSC | CD105 | 9.4 | 45.6 |
| | HLA-ABC | 10.5 | 73.6 |
| | CD105 + HLA-ABC | 0.6 | 35.9 |
| | HLA-DR + HLA-ABC | 4.7 | 14.5 |
| | CD105 + HLA-DR | 7.4 | 4.8 |
| | CD44 | 3.7 | 98.4 |
| | CD29 | 1.3 | 55.2 |
| | CD44+CD29 | 0.5 | 41 |
| | HLA-DR+CD29 | 0.1 | 4.4 |
| | HLA-DR+CD44 | 2.4 | 5.1 |
| | HLA-DR | 10.1 | 9.6 |
| | CD3 | 7 | 2.8 |
| | CD14 | 15 | 16.7 |
| | CD19 | 5.5 | 5.5 |

### SCs expansion after 10 days culture from frozen cells from placenta and cord-blood

To determine whether cells derived from CB and placenta, cultured in HMF-SC for 10 days and frozen in DMSO 10% or Glycerol 10% freezing solution, once thawed, maintain their original phenotype, the phenotype of SCs after thawing and culturing for 24h was analysed by FACS.

6 different vials were analysed:
**1.** CB-derived HSCs, frozen in DMSO
**2.** CB-derived HSCs, frozen in DMSO
**3.** Placenta-derived HSCs, frozen in DMSO
**4.** Placenta-derived HSCs, frozen in Glycerol
**5.** CB-derived HSCs, frozen in DMSO (2^{nd} freezing/thawing cycle)
**6.** Placenta-derived HSCs, frozen in DMSO (3^{rd} freezing/thawing cycle)

After thawing, all the cells maintained their HSC or MSC phenotype and kept high proliferation levels (CD117⁺).

T-cell contamination (CD3⁺ cells) decreased (under 5%); B-cell contamination (CD19⁺ cells) was less than 15% and monocyte contamination (CD14⁺ cells) strongly decreased (under 7.5%). Indeed, frozen cells re-cultured showed less contamination in comparison with fresh-isolated cells.

**Table 3A. Analysis of the phenotype of HSCs derived from CB or placenta, frozen in DMSO or in Glycerol, thawed and re-cultured with HMF-SC, added up with HMF-GF mix.**

| | **CB** | **CB** | **Placenta** |
|---|---|---|---|
| | **(DMSO)** | **(DMSO)** | **(DMSO)** |
| **ABC-G2** | 20 | 23 | 22.8 |
| **ABC-G2+CD34** | 19.4 | 1,7 | 19.3 |
| **ABC-G2+CD133** | 19.8 | 20.3 | 22.1 |
| **CD34** | 23.5 | 3.6 | 44.2 |
| **CD133** | 33.3 | 29.4 | 33.8 |
| **CD34+CD133** | 21.8 | 2.7 | 32.1 |
| **CD117** | 65.1 | 55.9 | 58.8 |
| **CD36** | 17.5 | 4.9 | 6.7 |
| **GPA** | 3.9 | 1.8 | 2.9 |
| **CD3** | 0.1 | 0.5 | 4.6 |
| **CD14** | 0 | 6.5 | 7 |
| **CD19** | 0 | 11.4 | 14.3 |
| **HLA-DR** | 0 | 11.1 | 13.4 |

| | **Placenta** | **CB** | **CB** |
|---|---|---|---|
| | **(Glycerol)** | **(Glycerol)** | **(DMSO)** |
| **ABC-G2** | 16.5 | 33.6 | 37.6 |
| **ABC-G2+CD34** | 12.7 | 30.5 | 31.8 |
| **ABC-G2+CD133** | 11.5 | 22.7 | 24.4 |
| **CD34** | 41.4 | 24.3 | 28.5 |
| **CD133** | 35.9 | 37.6 | 36.2 |
| **CD34+CD133** | 32.8 | 21.9 | 25.3 |
| **CD117** | 56.4 | 45.6 | 42.1 |
| **CD36** | 2.9 | 2.1 | 0.6 |
| **GPA** | 1.1 | 0.4 | 0.3 |
| **CD3** | 0.9 | 0.1 | 0 |
| **CD14** | 1.8 | 0.4 | 0 |
| **CD19** | 0 | 0.3 | 0.3 |
| **HLA-DR** | 10.4 | 9.6 | 11.2 |

**Table 3B. Analysis of the phenotype of MSCs derived from CB or placenta, frozen in DMSO or in Glycerol, thawed and re-cultured with HMF-SC, added up with HMF-GF mix.**

| | **CB** | **CB** | **Placenta** |
|---|---|---|---|
| | **(DMSO)** | **(DMSO)** | **(DMSO)** |
| **CD105** | 42 | 43 | 51 |
| **HLA-ABC** | 71.4 | 68.4 | 70.9 |
| **CD105 + HLA-ABC** | 40.3 | 42.3 | 50.8 |
| **HLA-DR + HLA-ABC** | 11.1 | 3.8 | 12.5 |
| **CD105 + HLA-DR** | 9.3 | 1.5 | 2.1 |
| **CD44** | 98.6 | 99.7 | 96.2 |
| **CD29** | 62.9 | 66.4 | 56.2 |
| **CD44+CD29** | 55.2 | 53.9 | 43.1 |
| **HLA-DR+CD29** | 3.9 | 1.8 | 2.9 |
| **HLA-DR+CD44** | 1.2 | 3.7 | 2.5 |
| **HLA-DR** | 13 | 4.8 | 3.6 |
| **CD3** | 0.4 | 2.8 | 0 |
| **CD19** | 10.6 | 12.7 | 2.3 |
| **CD14** | 1.7 | 3.7 | 1.9 |

| | **Placenta** | **CB** | **CB** |
|---|---|---|---|
| | **(Glycerol)** | **(Glycerol)** | **(DMSO)** |
| **CD105** | 51 | 31 | 29 |
| **HLA-ABC** | 57.3 ' | 61.9 | 55.4 |
| **CD105 + HLA-ABC** | 44.3 | 26.8 | 18.2 |
| **HLA-DR + HLA-ABC** | 1.9 | 10.7 | 20.6 |
| **CD105 + HLA-DR** | 3.7 | 1.4 | 11.3 |
| **CD44** | 91.4 | 99.2 | 98.6 |
| **CD29** | 71.9 | 53.4 | 62.8 |
| **CD44+CD29** | 70 | 51.3 | 60.4 |
| **HLA-DR+CD29** | 3.9 | 1.8 | 2.9 |
| **HLA-DR+CD44** | 0.4 | 1.3 | 1.7 |
| **HLA-DR** | 4.9 | 7.8 | 17.6 |
| **CD3** | 1.4 | 0 | 0 |
| **CD19** | 4.3 | 2.1 | 0.9 |
| **CD14** | 3.8 | 3.7 | 5.4 |

No significant differences between the outcomes of cells frozen in DMSO or in Glycerol or after more than one freezing/thawing cycle could be observed (Table 3).

### SCs expansion after 10 days culture from fresh cells, isolated from cord-blood, in presence of different media and GF mixtures

The outcomes in HSCs expansion from CB obtained with the use of HMF-SC and HMF-GF mix; with StemSpan and standard GF mix; and with their combinations were compared (Fig. 1).

It could be shown that cells cultured with HMF-SC and HMF-GF mix proliferated much better than:
- Cells expanded in StemSpam + standard GF mix (less cells and more apoptosis);
- Cells grown in StemSpam + HMF-GF mix (less cells, due to decreased cell proliferation; more clusters; low levels of apoptosis);
- Cells grown in HMF-SC + standard GF mix (mildly increased apoptosis).

### SCs expansion after 10 days culture from fresh cells, isolated from cord-blood in presence of variants of RMF-SC

During the development of HMF-SC, several combinations of factors were tried, in order to determine which were the most important for SCs *in vitro* expansion without major apoptotic events and with high rate of SCs proliferation. Moreover, these cocktails were as well under investigation for their capability to trigger the apoptosis of contaminant cells i.e. lymphocytes, erythrocytes, granulocytes, platelets, while supporting the expansion of the SCs. The developing of HMF-SC went through different phases. In the following description, some of the most important steps of its development are reported. In all the experiments, the following GF mix was used:

| | |
|---|---|
| SCF | 100 µg/mL |
| GM-SCF | 3.5 µg/mL |
| TPO | 7 µg/mL |
| Flt-3 | 20 µg/mL |

The original composition of HMF-SC includes the following ingredients:

| | |
|---|---|
| Iscove medium | To 500 mL |
| Bovine serum albumin | 0.05% w/V |
| Human Insulin | 0.5 mM |
| Sodium Pyruvate | 0.1 mM |
| L-Glutamine | 2 mM |
| 2-mercaptoethanol | 0.01% V/V |
| IGF-1 | 0.4 mM |

In this case, SCs could be expanded for 10 days before going into apoptosis and in trans-differentiation towards hematopoietic cells. The max amount of SCs, expanded in this way was about 10*10⁶ with a contamination by lymphocytes over 50%. Anyhow, it was possible to trasdifferentiate the cells into erythroid precursors, which can survive about 10 days without spontaneously differentiate or going into apoptosis. The max amount of erythroid precursor was about 25*10⁶ and they could completely differentiate into erythrocytes without showing major apoptotic events. The medium added up with the GF mix was stable for 4 days at 4°C. After a cycle of freezing/thawing (in FBS 90% and DMSO 10%) the apoptotic rate was about 30% and the phenotype of the SCs was not steady kept. After 2 additional days in culture, the cells tended to differentiate into erythroid precursors.

Thereafter, the original composition underwent the following changes:

| | |
|---|---|
| Iscove medium | To 500 mL |
| Bovine serum albumin | 0.05% w/V |
| Selenium-Transferrin-Insulin | 50 mL |
| Sodium Pyruvate | 0.1 mM |
| L-Glutamine | 2 mM |
| Non essential a.a. | 100 µg/mL |

In this case, the SCs could be expanded for 2 weeks before going into apoptosis and in trans-differentiation towards hematopoietic cells. The max amount of SCs, expanded in this way was about 30*10⁶ with a contamination by lymphocytes around 30%. Anyhow, it was possible to transdifferentiate the cells into erythroid precursors, which can survive about 30 days without spontaneously differentiating or going into apoptosis. The max amount of erythroid precursor was about 7*10⁷ and they could completely differentiate into erythrocytes without showing major apoptotic events. The medium added up with the GF mix was stable for 1 week at 4°C. After a cycle of freezing/thawing (in FBS 90% and DMSO 10%) the apoptotic rate was about 22% and the phenotype of the SCs was maintained by around 60% of the cells. The cell contamination was lowered to 15%. After 5 additional days in culture, the cells tended to differentiate into erythroid precursors.

The subsequent modification of the SCs medium brought the following changes:

| | |
|---|---|
| Iscove medium | To 500 mL |
| Selenium-Transferrin-Insulin | 50 mL |
| Sodium Pyruvate | 0.1 mM |
| L-Glutamine | 2 mM |
| Nucleoside | 10 µg/mL |
| Non essential a.a. | 100 µg/mL |

In this case, the SCs could be expanded for 10 days before going into apoptosis and in trans-differentiation towards hematopoietic cells. The max amount of SCs, expanded in this way was about 5-7*10⁷ with a contamination by lymphocytes around 25%. Anyhow, it was possible to transdifferentiate the cells into erythroid precursors, which can survive about 50 days without spontaneously differentiating or going into apoptosis. The max amount of erythroid precursor was about 1*10⁸ and they could completely differentiate into erythrocytes without showing major apoptotic events. The medium added up with the GF mix was stable for 1 week at 4°C. After a cycle of freezing/thawing (in FBS 90% and DMSO 10%) the apoptotic rate was about 15% and the phenotype of the SCs was maintained by around 80% of the cells. The cell contamination was lowered to 10%. After 10 additional days in culture, the cells tended to differentiate into erythroid precursors.

For an overview of these results it is referred to Table 4

**Table 4. Analysis of the phenotype of fresh-cultured HSCs and MSC, derived from CB (R1-4), or frozen in DMSO (R1-4 after R), cultured in 4 different (1-4) developing versions of HMF-SCs, in presence of HMF-GF mix.**

| | | | | | **R1 after** | **R2 after** | **R3 after** | **RF after** |
|---|---|---|---|---|---|---|---|---|
| | **R1** | **R2** | **R3** | **RF** | **F** | **F** | **F** | **F** |
| **CD117** | 12.7 | 42.3 | 33.7 | 56.7 | 4.7 | 23.7 | 37.9 | 55.9 |
| **ABC-G2+CD117** | 0.4 | 0.7 | 2.6 | 2.3 | 0.4 | 0.1 | 2.8 | 2 |
| **CD34** | 31.2 | 42.8 | 61.2 | 67.4 | 11.4 | 34.6 | 55.8 | 69.8 |
| **ABC-G2+CD34** | 0.2 | 0.1 | 0.1 | 2.1 | 0.2 | 0.3 | 0.3 | 1.8 |
| **CD34+CD117** | 5.9 | 22.4 | 11.7 | 52.3 | 3.2 | 11.3 | 4.8 | 53.5 |
| **SSEA-4+CD34** | 0.2 | 0.4 | 0.2 | 1.9 | 0.1 | 0.1 | 0.1 | 1.6 |
| **CD133** | 0.7 | 2.1 | 0.2 | 53.2 | 0.3 | 1.7 | 0.3 | 49.8 |
| **CD117+CD133** | 0.2 | 2 | 0.1 | 50.7 | 0.1 | 0.8 | 0.3 | 48.2 |
| **CD34+CD133** | 0.2 | 2.1 | 0.2 | 32.7 | 0.3 | 0.3 | 0.1 | 34.9 |
| **CD34+HLA-DR** | 21.5 | 17.6 | 10.3 | 15.4 | 10.4 | 25.7 | 11.2 | 18.7 |
| **CD34+HLA-ABC** | 24.3 | 37.6 | 39.7 | 65.3 | 5.9 | 21.1 | 43.2 | 67.9 |
| **SSEA-4+ABC-G2** | 0.1 | 0.2 | 0.1 | 2.3 | 0.1 | 0.1 | 0.2 | 2.9 |
| **CD133+ABC-G2** | 0.2 | 0.2 | 0.2 | 0.9 | 0.3 | 0.1 | 0.1 | 1.4 |
| **CD117+SSEA-4** | 0.2 | 0.1 | 0.1 | 0.7 | 0.1 | 0.1 | 0.1 | 1.2 |
| **CD9+CD34** | 11.4 | 14.3 | 23.4 | 36.8 | 4.3 | 3.5 | 11.1 | 28.7 |
| **CD34+CD166** | 9.3 | 8.7 | 15.4 | 39.8 | 3.8 | 2.9 | 4.3 | 31.8 |
| **CD10+CD34** | 2.8 | 4.5 | 2.9 | 21.3 | 0.8 | 2.9 | 0.4 | 18.7 |
| **CD34+CD105** | 3.9 | 4.9 | 22.9 | 39.7 | 0.9 | 2.1 | 13.2 | 29.8 |
| **CD29+CD34** | 1.4 | 0.9 | 19.8 | 41.2 | 0.3 | 0.1 | 10.8 | 33.9 |
| **CD34+CD73** | 1.3 | 0.1 | 17.6 | 40.7 | 0.2 | 0.4 | 21.8 | 43.9 |
| **CD13+CD34** | 1.9 | 0.3 | 2.1 | 38.2 | 0.1 | 0.4 | 0.9 | 38.6 |
| **SSEA-4** | 0.5 | 0.8 | 0.4 | 2.4 | 0.1 | 0.8 | 0.9 | 1.3 |
| **ABC-G2** | 0.8 | 1.6 | 2.3 | 2.9 | 0.1 | 0.4 | 1.1 | 2.1 |
| **HLA-DR** | 39.8 | 29.4 | 31.2 | 18.9 | 42.7 | 35.5 | 39.7 | 16.4 |
| **CD44** | 92.3 | 97.3 | 96.3 | 91.4 | 95.9 | 91.8 | 89.9 | 93.8 |
| **CD29** | 24.3 | 39.8 | 43.7 | 69.8 | 10.5 | 21.1 | 54.3 | 56.9 |
| **CD44+CD29** | 21.6 | 31.2 | 42.1 | 28.9 | 8.4 | 11.7 | 29.8 | 15.9 |
| **CD90** | 11.2 | 56.2 | 78.3 | 75.7 | 10.3 | 32.9 | 61.2 | 68.1 |
| **CD90+CD29** | 18.1 | 33.2 | 41.1 | 47.9 | 2.3 | 21 | 33.8 | 38.6 |
| **CD44+CD90** | 3.9 | 50.3 | 70.9 | 41.2 | 2.1 | 22.9 | 60.8 | 47.8 |
| **CD105** | 10.6 | 16.8 | 32.3 | 37.2 | 5.9 | 9.7 | 24.1 | 33.9 |
| **HLA-ABC** | 23.2 | 45.9 | 89.6 | 96.5 | 18.7 | 30.1 | 71.2 | 91.7 |
| **CD105+HLA-ABC** | 9.3 | 11.3 | 31 | 31.4 | 1.1 | 8.1 | 20.6 | 31.8 |
| **HLA-DR+HLA-ABC** | 20.3 | 15.3 | 29.4 | 18.9 | 10.2 | 11.1 | 33.9 | 15.3 |
| **HLA-DR+CD105** | 10.1 | 9.3 | 2,1 | 3.9 | 5.4 | 5.2 | 20.1 | 3.8 |
| **CD166** | 10.2 | 42.9 | 69.8 | 54.3 | 4.8 | 67.4 | 55.9 | 55.1 |
| **CD166+CD9** | 2.5 | 8.9 | 31.2 | 6.5 | 1.1 | 3.4 | 2.7 | 7.9 |
| **CD9** | 15.7 | 23.4 | 46.8 | 41.9 | 7.9 | 10.8 | 20.5 | 54.2 |
| **CD10** | 9.3 | 39.8 | 2.1 | 34.7 | 5.5 | 48.9 | 1.1 | 32.9 |
| **CD9+CD10** | 1.2 | 21.4 | 1.1 | 3.2 | 1.3 | 2.3 | 0.7 | 1.1 |
| **CD10+CD166** | 5.9 | 34.9 | 1.9 | 23.6 | 1.1 | 23.5 | 0.3 | 15.4 |
| **CD73** | 4.5 | 89.4 | 28.8 | 87.6 | 6 | 91.2 | 43.2 | 73.2 |
| **CD73+CD29** | 4.1 | 33.1 | 19.2 | 28.1 | 1.1 | 19 | 10.4 | 36.8 |
| **CD13** | 3.9 | 54.8 | 21.2 | 55.2 | 4.2 | 19.2 | 13.2 | 54.1 |
| **CD13+CD29** | 1.1 | 30.1 | 2.9 | 64.3 | 0.6 | 10.8 | 1.1 | 73.9 |
| **CD13+CD73** | 0.9 | 29.9 | 2.8 | 63.2 | 0.1 | 12.3 | 1.9 | 65.4 |
| **CD45** | 78.9 | 89.4 | 89.7 | 43.9 | 81.4 | 54.8 | 94.3 | 53.6 |
| **CD19** | 34.8 | 2.1 | 4.5 | 3.4 | 11.5 | 3.4 | 5.1 | 2.1 |
| **CD3** | 54.3 | 22.1 | 17.9 | 6.9 | 34.6 | 20.9 | 24.6 | 3.4 |
| **CD14** | 68.9 | 27.7 | 21.3 | 18.9 | 76.3 | 30.5 | 13.2 | 23.5 |

### SCs differentiation into erythroid cells

To determine the transdifferentiation potentials of HSCs, cultured in HMF-SC, erythroid differentiation upon addition of 10 U/mL Epo, 10 ng/mL insulin, 3x10⁻⁶ M ZK112.993, and 1 mg/mL iron-saturated human transferrin (GF1) or of 100 ng/mL IL-3, 200 U/mL GM-CSF and 5U/mL Epo (GF2) was induced and the levels of erythrocyte maturation by analysing hemoglobin accumulation were compared. Within 7 days and with a partial medium change every second day, HSCs fully differentiated into enucleated erythrocytes and produced high amounts of hemoglobin (Fig. 2), even slightly higher than cells differentiated in StemSpam and GF1 (Control).

Additional data about erythroid cells will be included in a publication (all data available with the exception of some WB, regarding signal transduction upon incubation with hormones).

### Summary of the example:

The present example provides a medium to expand SCs from cord-blood, placenta and amniotic fluid.

It could be shown that in this medium both HSCs and MSCs as well as fibroid-like cells (amniotic cells) can be expanded to obtain a minimum of 50*10⁶ cells without the need of enrichment steps.

It could also be shown that the HSCs maintain their phenotype also after freezing in DMSO and in Glycerol. Contaminations with other cells, such as T-cells, B-cells and monocytes were always kept low level.

HSCs can be as well differentiated in erythroid cells, following different protocols.

## Claims

1. A growth medium for in vitro stem cell expansion comprising:
| | |
|---|---|
| selenium-transferrin-insulin | 1% to 20%, |
| Pyruvate, preferably sodium pyruvate, | 0.05 to 1 mM, |
| L-glutamine | 0.5 to 10 mM, |
| nucleosides | 1 to 100 µg/ml, |
| at least one amino acid, preferably non-essential amino acid | 5 to 1000 µg/ml, |
Iscove's modified Dulbecco's medium (IMDM) up to 100%.

2. Medium according to claim 1, **characterised in that** the medium comprises further LDL, preferably human LDL, in an amount of 1 to 100 µg/ml.

3. Medium according to claim 1 or 2, **characterised in that** the medium comprises further an iron salt, preferably ferrous sulphate, in an amount of 1 to 100 nM.

4. Medium according to any one of claims 1 to 3, **characterised in that** the medium comprises:
| | |
|---|---|
| Iscove medium | to 500 mL |
| Selenium-Transferrin-Insulin | 50 mL |
| Sodium Pyruvate | 0.1 mM |
| L-Glutamine | 2 mM |
| Nucleosides | 10 µg/mL |
| Non essential amino acids | 100 µg/mL |

5. Medium according to any one of claims 1 to 4, **characterised in that** the medium comprises:
| | |
|---|---|
| Iscove medium | to 500 mL |
| Selenium-Transferrin-Insulin | 50 mL |
| human LDL | 40 µg/mL |
| Sodium Pyruvate | 0.1 mM |
| L-Glutamine | 2 mM |
| Iron Sulphate | 40 nM |
| Nucleosides | 10 µg/mL |
| Non-essential amino acids | 100 µg/mL |

6. Use of a medium according to any one of claims 1 to 5 for cultivating hematopoietic, amniotic and/or mesenchymal stem cells.

7. Method for cultivating hematopoietic, amniotic and/or mesenchymal stem cells comprising the steps:
- providing a stem cell source, and
- incubating said source with medium according to any one of claims 1 to 5.
